# EUROPEAN PATENT APPLICATION

(11) **EP 3 900 632 A1**
(43) Date of publication of application: **27.10.2021**
(21) Application number: 18943361.8
(22) Date of filing: 18.12.2018
(51) Int. Cl.: A61B 5/20, G01N 33/493

(54) **URINE QUANTITY MEASUREMENT DEVICE**

(71) Applicant: Horiguchi, Akira, Takarazuka-shi, Hyogo, 665-0061 (JP); Yoshino, Kazutaka, Kobe-shi, Hyogo, 651-1515 (JP)
(72) Inventor: Horiguchi, Akira, Takarazuka-shi, Hyogo, 665-0061 (JP); Yoshino, Kazutaka, Kobe-shi, Hyogo, 651-1515 (JP)
(74) Representative: Viering, Jentschura & Partner mbB Patent- und Rechtsanwälte
(86) International application number: PCT/JP2018/046601
(87) International publication number: WO 2020/129158

(57) **Abstract**

A urine quantity measurement device (100) includes a suspension section (4), a sensor, a controller, a housing (30), and an attachment section (2). A urine collection container (C) is suspended from the suspension section (4). The urine collection container (C) is for collecting urine from a person (P) lying on a bed (B). The sensor outputs a signal indicating a weight of the urine collection container (C) based on a force acting on the suspension section (4) from the urine collection container (C). The controller generates output data based on the signal. The housing (30) houses the sensor and the controller. The attachment section (2) is arranged on the bed (B) and supports the housing (30). The output data indicates the weight of the urine collection container or a quantity of the urine collected in the urine collection container.

## Description

### [TECHNICAL FIELD]

The present invention relates to a urine quantity measurement device.

### [BACKGROUND ART]

Patent Literature 1 describes a balanced hydration system which measures a urine quantity. The balanced hydration system disclosed in Patent Literature 1 includes a unit and a hook. A urine collection bag is hung from the hook. The unit measures the weight of urine collected in the urine collection bag.

### [CITATION LIST]

### [Patent Literature]

[Patent Literature 1]
Japanese Patent Application Laid-Open Publication No. 2010-506613

### [SUMMARY OF INVENTION]

### [Technical Problem]

However, the balanced hydration system disclosed in Patent Literature 1 is used while mounted on an intravenous (IV) pole. The IV pole is arranged at the side of a bed. Accordingly, an arrangement space in which the IV pole is to be arranged is needed at the side of the bed in order to introduce the balanced hydration system disclosed in Patent Literature 1.

The present invention takes into account the above problem, and an objective thereof is to provide a urine quantity measurement device capable of measuring a urine quantity without need of an arrangement space provided at the side of a bed.

### [Solution to Problem]

A urine quantity measurement device disclosed in the present application includes a suspension section, a sensor, a controller, a housing, and an attachment section. A urine collection container is suspended from the suspension section. The urine collection container is for collecting urine from a person lying on a bed. The sensor outputs a signal indicating a weight of the urine collection container based on a force acting on the suspension section from the urine collection container. The controller generates output data based on the signal. The housing houses the sensor and the controller. The attachment section is arranged on the bed. The attachment section supports the housing. The output data indicates the weight of the urine collection container or a quantity of the urine collected in the urine collection container.

The urine quantity measurement device of the present invention further includes a restricting section. The restricting section restricts a posture of the urine quantity measurement device such that a pulling force from the urine collection container is caused to act on the sensor in a vertical direction.

In the urine quantity measurement device of the present invention, the attachment section includes a base, a housing support section, and a first joint. The base is arranged on the bed. The housing support section supports the housing. The first joint joins the base to the housing support section.

In the urine quantity measurement device of the present invention, the restricting section includes a first restricting section. The first joint changes a posture of the housing support section relative to the base. The first restricting section is composed by the first joint.

In the urine quantity measurement device of the present invention, the suspension section includes a base end, a holding section, and a second joint. The base end is connected to the sensor. The urine collection container is suspended from the holding section. The second joint joins the base end to the holding section.

In the urine quantity measurement device of the present invention, the restricting section includes a second restricting section. The second joint changes a posture of the holding section relative to the base end. The second restricting section is composed by the second joint.

In the urine quantity measurement device of the present invention, the sensor has a beam extending in a direction intersecting with a vertical direction. The base end extends from the beam in a direction intersecting with the direction in which the beam extends. The holding section is provided on an opposite side of the beam from a side to which the base end is connected.

In the urine quantity measurement device of the present invention, the holding section is provided as a plurality of holding sections.

In the urine quantity measurement device of the present invention, the sensor has a first beam extending in a direction intersecting with the vertical direction. The second joint includes a second beam. The base end extends from the first beam in a direction intersecting with the direction in which the first beam extends. The second beam is provided on an opposite side of the first beam from a side to which the base end is connected. The plurality of holding sections are provided in positions on the second beam at which forces acting from the urine collection container are uniform.

A urine quantity measurement device of the present invention includes a suspension section, a sensor, a controller, a housing, an attachment section and an output section. A urine collection container is suspended from the suspension section. The urine collection container is for collecting urine from a person lying on a bed. The sensor outputs a signal indicating a weight of the urine collection container based on a force acting on the suspension section from the urine collection container. The controller generates output data based on the signal. The housing houses the sensor and the controller. The attachment section is arranged on the bed and supports the housing. The output section outputs the output data. The sensor includes a beam extending in a direction intersecting with a vertical direction. The suspension section includes a base end and a holding section. The base end extends from the beam in a direction intersecting with the direction in which the beam extends. The holding section is provided on an opposite side of the beam from a side to which the base end is connected. The urine collection container is suspended by the holding section. The output data indicates the weight of the urine collection container or a quantity of urine collected in the urine collection container.

A urine quantity measurement device of the present invention includes a suspension section, a sensor, a controller, a housing, an attachment section, and an output section. A urine collection container is suspended from the suspension section. The urine collection container is for collecting urine from a person lying on a bed. The sensor outputs a signal indicating a weight of the urine collection container based on a force acting on the suspension section from the urine collection container. The controller generates output data based on the signal. The housing houses the sensor and the controller. The attachment section is arranged on the bed and supports the housing. The output section outputs the output data. The sensor includes a first beam extending in a direction intersecting with a vertical direction. The suspension section includes a base end, a second beam, and a plurality of holding sections. The base end extends from the first beam in a direction intersecting with the direction in which the first beam extends. The second beam is provided on an opposite side of the first beam from a side to which the base end is connected. The plurality of holding sections are provided in positions on the second beam at which forces acting from the urine collection container are uniform. The urine collection container is suspended by the plurality of holding sections. The output data indicates the weight of the urine collection container or a quantity of the urine collected in the urine collection container.

### [Advantageous Effects of Invention]

According to the urine quantity measurement device of the present invention, a urine amount can be measured without need of providing an arrangement space at the side of a bed.

### [BRIEF DESCRIPTION OF DRAWINGS]

[FIG. 1]

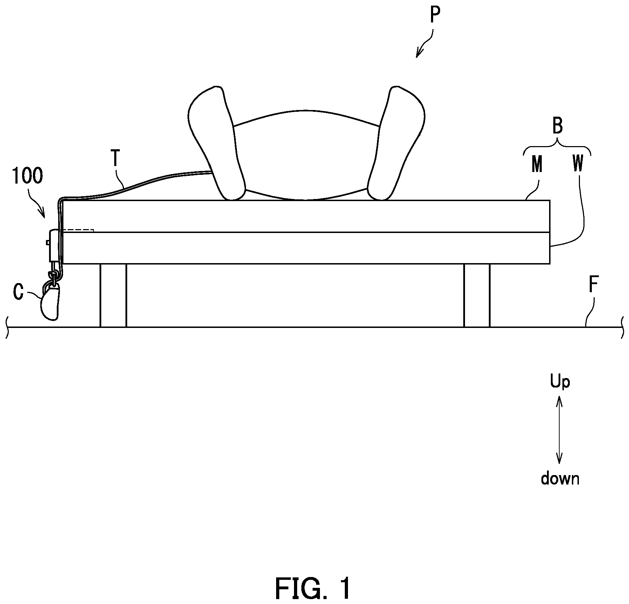

   FIG. 1 is a diagram illustrating a use state of a urine quantity measurement device according to a first embodiment of the present invention.
[FIG. 2]

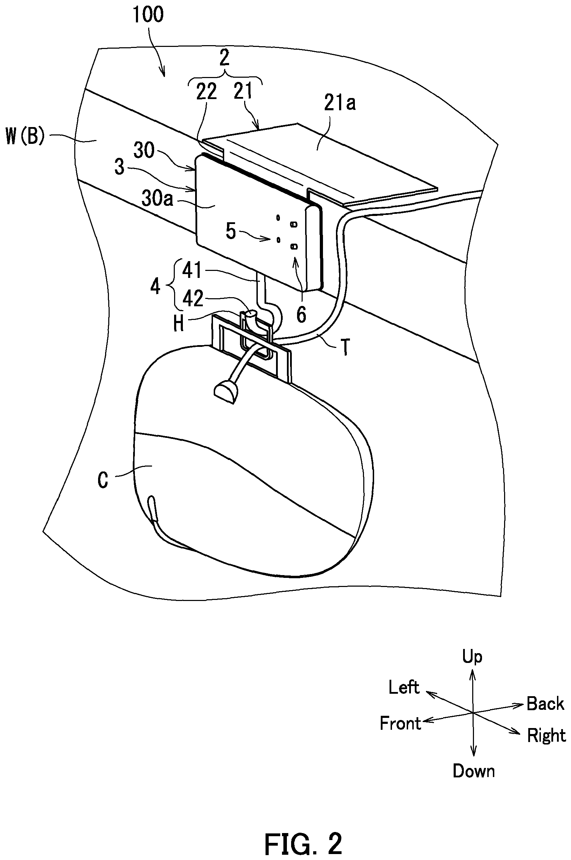

   FIG. 2 is a diagram illustrating a configuration of the urine quantity measurement device according to the first embodiment of the present invention.
[FIG. 3]

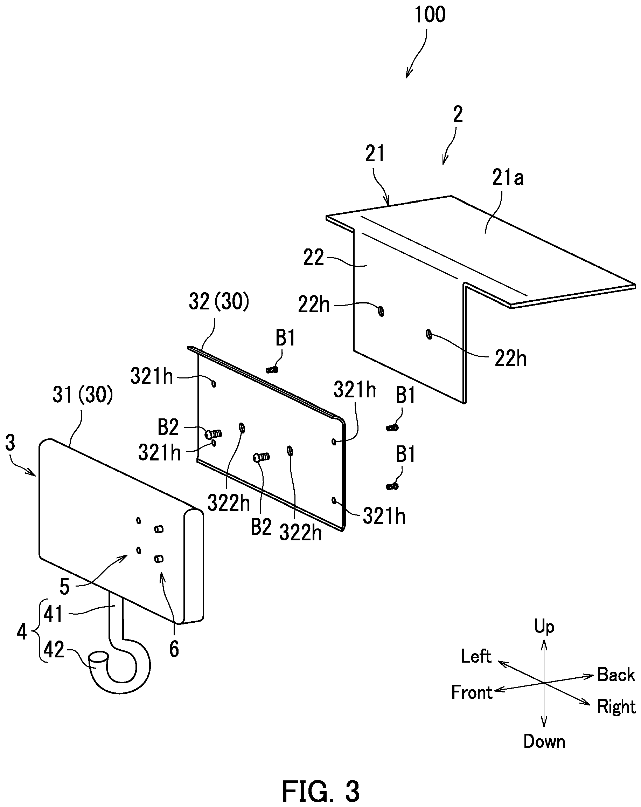

   FIG. 3 is an exploded perspective view of the urine quantity measurement device according to the first embodiment of the present invention.
[FIG. 4]

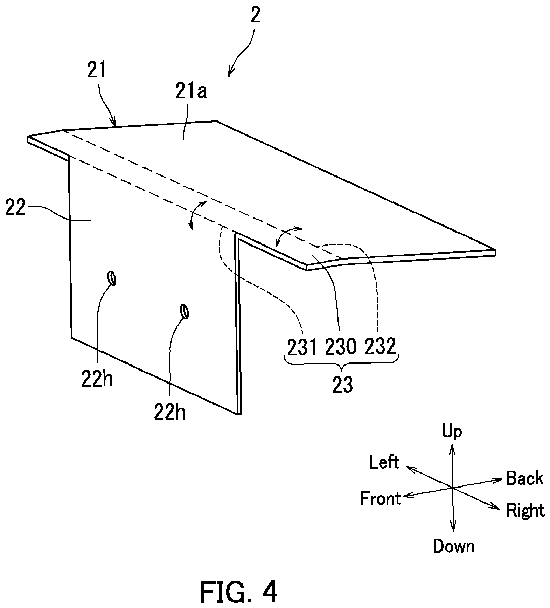

   FIG. 4 is a diagram illustrating a configuration of an attachment section according to the first embodiment of the present invention.
[FIG. 5]

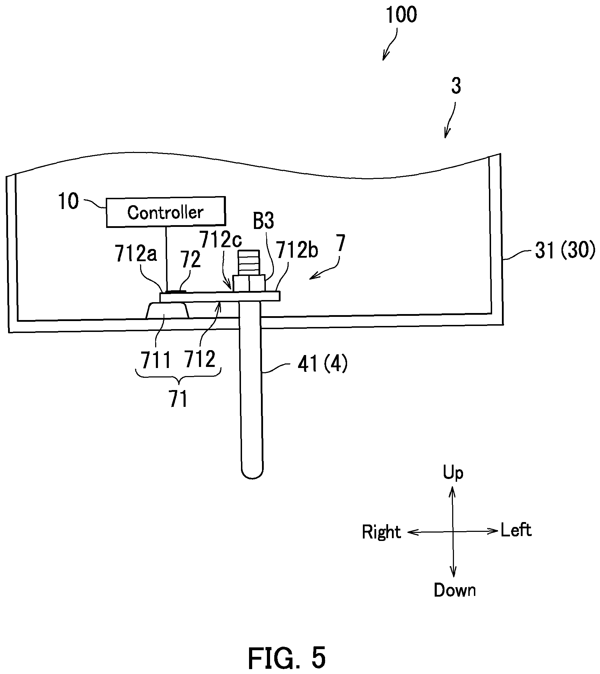

   FIG. 5 is a diagram illustrating a suspension section and a vicinity thereof according to the first embodiment of the present invention.
[FIG. 6]

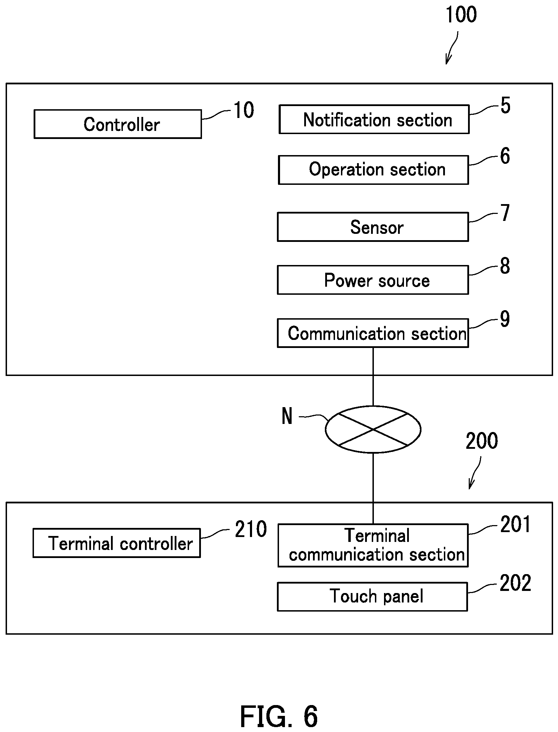

   FIG. 6 is a block diagram illustrating the configuration of the urine quantity measurement device according to the first embodiment of the present invention.
[FIG. 7]

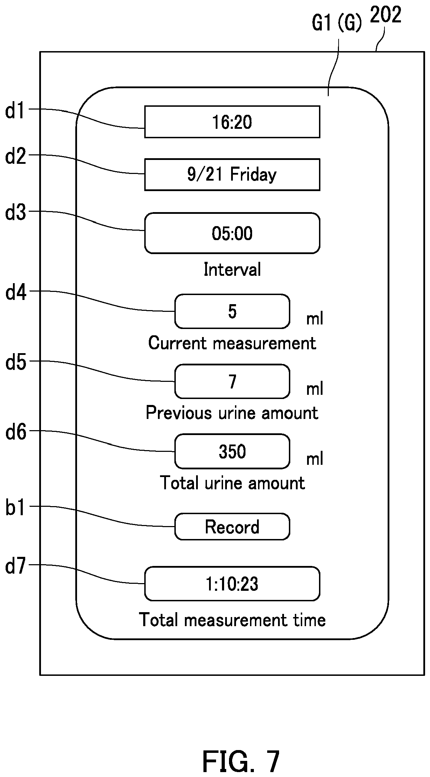

   FIG. 7 is a diagram illustrating an example of a urine quantity measurement screen according to the first embodiment of the present invention.
[FIG. 8]

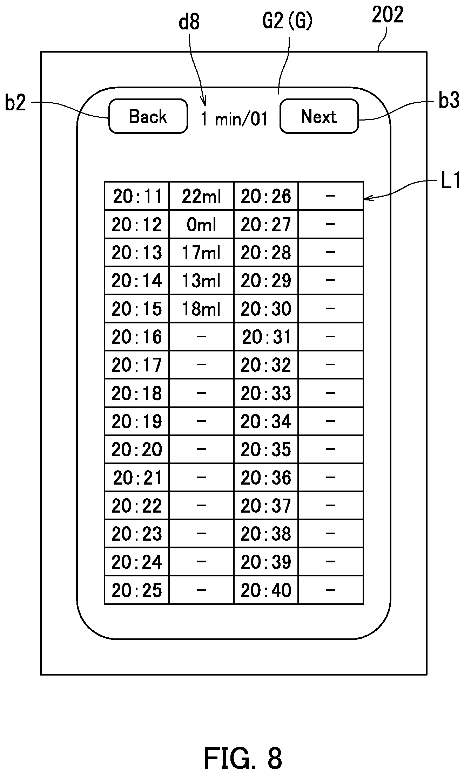

   FIG. 8 is a diagram illustrating an example of the urine quantity measurement screen according to the first embodiment of the present invention.
[FIG. 9]

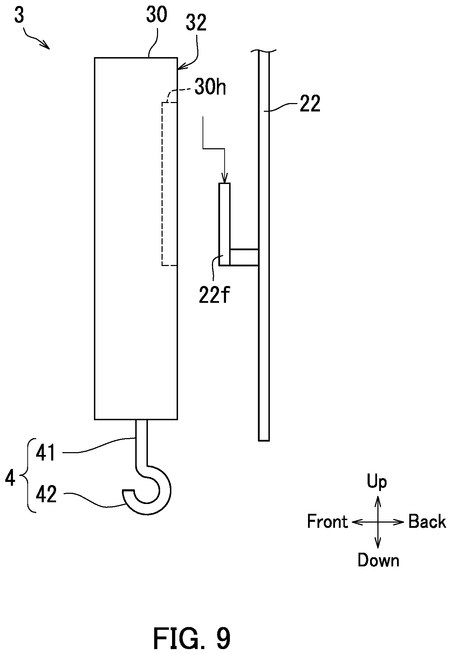

   FIG. 9 is a diagram illustrating an alternative example of a housing support section and a housing according to the first embodiment of the present invention.
[FIG. 10]

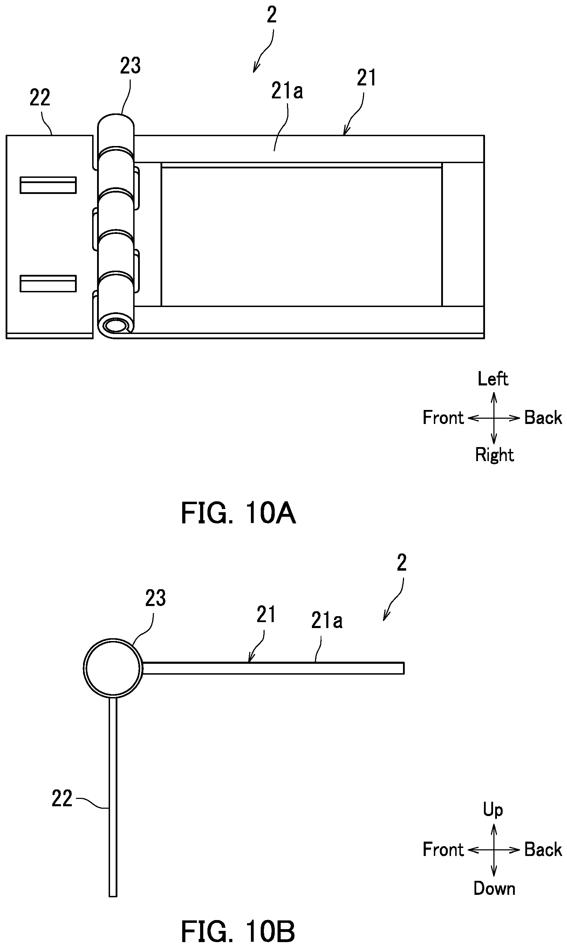

   FIG. 10A is a diagram illustrating a first alternative example of a first joint according to the first embodiment of the present invention. FIG. 10B is a diagram illustrating a second alternative example of the first joint according to the first embodiment of the present invention.
[FIG. 11]

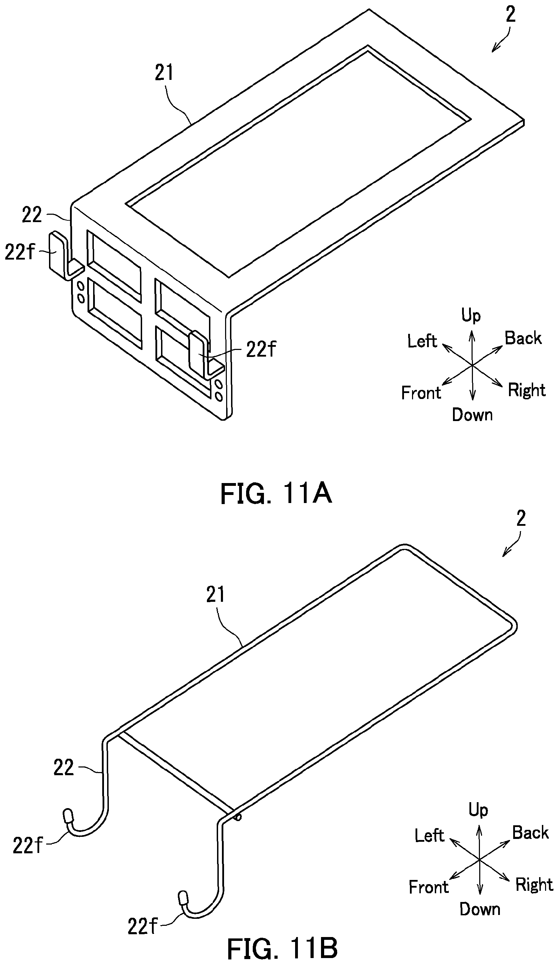

   FIG. 11A is a diagram illustrating a first alternative example of the attachment section according to the first embodiment of the present invention. FIG. 11B is a diagram illustrating a second alternative example of the attachment section according to the first embodiment of the present invention.
[FIG. 12]

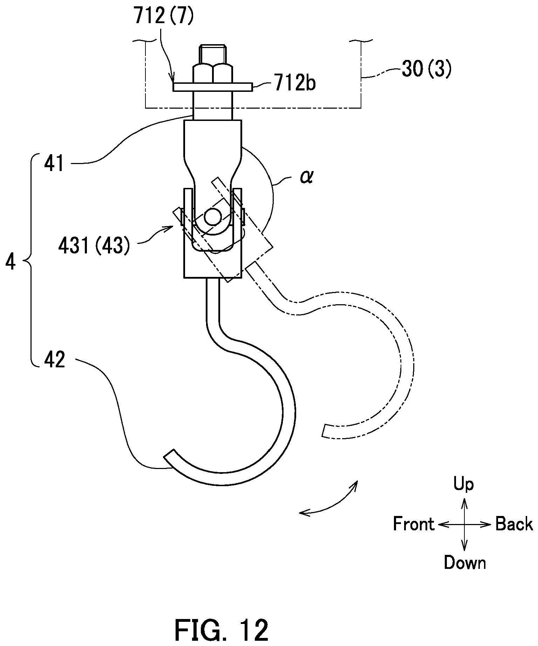

   FIG. 12 is a diagram illustrating a configuration of the suspension section according to a second embodiment of the present invention.
[FIG. 13]

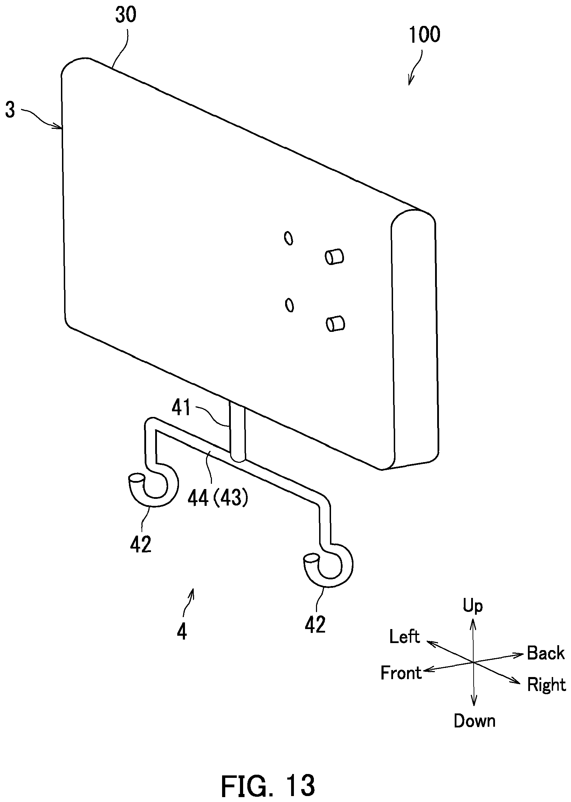

   FIG. 13 is a diagram illustrating a configuration of the urine quantity measurement device according to a third embodiment of the present invention.
[FIG. 14]

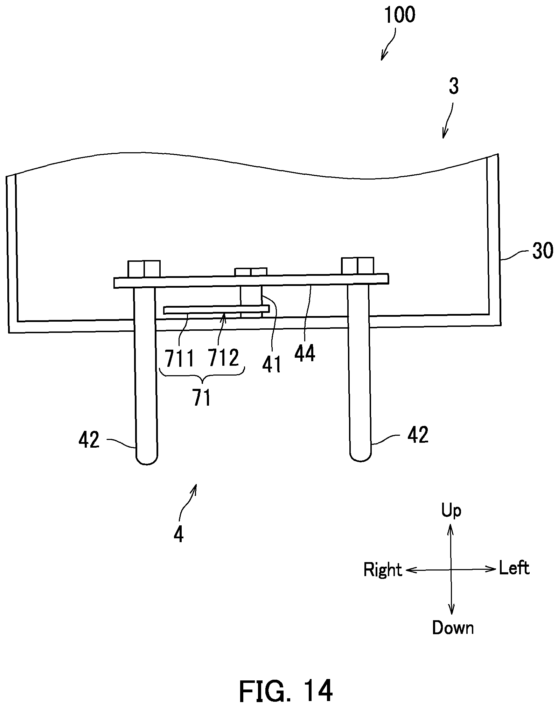

   FIG. 14 is a diagram illustrating a first alternative example of the suspension section according to the third embodiment of the present invention.
[FIG. 15]

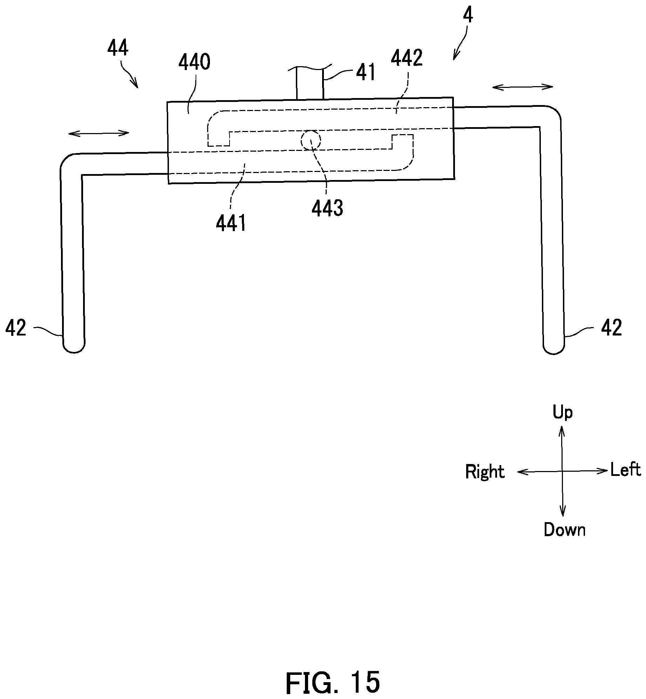

   FIG. 15 is a diagram illustrating a second alternative example of the suspension section according to the third embodiment of the present invention.
[FIG. 16]

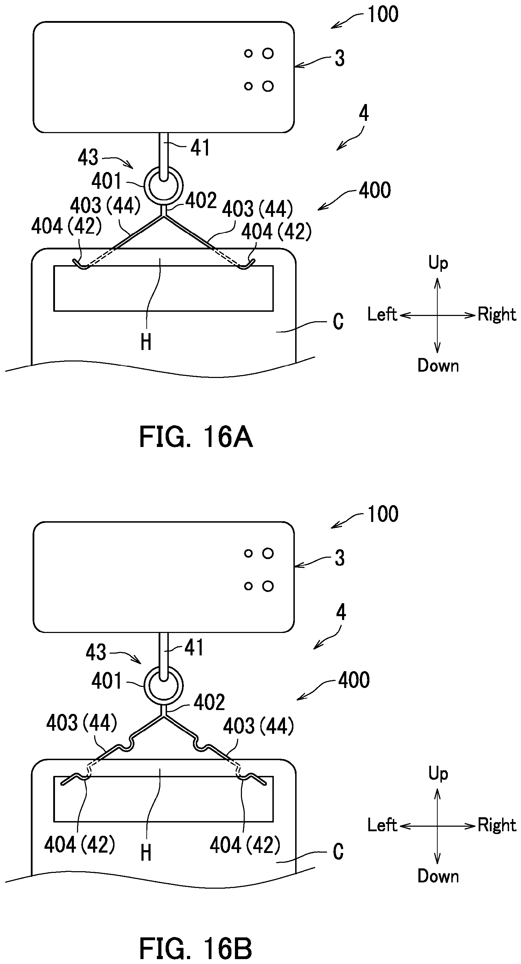

   FIG. 16A is a diagram illustrating the suspension section according to a fourth embodiment of the present invention. FIG. 16B is a diagram illustrating an alternative example of the suspension section according to the fourth embodiment of the present invention.

### [DESCRIPTION OF EMBODIMENTS]

The following describes a urine quantity measurement device according to embodiments of the present invention with reference to the accompanying drawings. It should be noted that elements which are the same or equivalent are labeled with the same reference signs in the drawings and description thereof is not repeated.

### [First Embodiment]

First, a use state of a urine quantity measurement device 100 according to a first embodiment is described with reference to FIG. 1. FIG. 1 is a diagram illustrating the use state of the urine quantity measurement device 100 according to the first embodiment.

As illustrated in FIG. 1, the urine quantity measurement device 100 is arranged on a bed B. The bed B is arranged on a floor F. In the present embodiment, the floor F is horizontal. In the following, the embodiment is described with a direction orthogonal to the floor F as an up-and-down direction. A side of the floor F on which the bed B is arranged is described as an "upper side" in the embodiment, and an opposite side thereto is described as a "lower side". In the present embodiment, a direction from the upper side to the lower side matches a vertical direction.

The bed B includes a frame W and a mattress M. The mattress M is supported by the frame W. A person P lays on the upper surface of the mattress M. The person P is a patient, for example.

The urine quantity measurement device 100 measures a urine quantity of the person P. In detail, the urine quantity measurement device 100 supports a urine collection container C. An end of a urine guide tube T is connected to the urine collection container C. The other end of the urine guide tube T is connected to a urinary catheter placed inside of the person P, for example. Urine from the person P is collected in the urine collection container C via the urine guide tube T. The urine quantity measurement device 100 measures a urine quantity of the person P by measuring the weight of the urine collection container C.

Next, a configuration of the urine quantity measurement device 100 according to the first embodiment is described with reference to FIG. 2. FIG. 2 is a diagram illustrating the configuration of the urine quantity measurement device 100 according to the first embodiment.

As illustrated in FIG. 2, the urine quantity measurement device 100 includes an attachment section 2, a main body 3, a suspension section 4, a notification section 5, and an operation section 6. The attachment section 2 includes paper as a material, for example. In the present embodiment, the attachment section 2 is formed by bending a single flat plate. Specifically, the attachment section 2 is formed by bending a single sheet of cardboard.

The attachment section 2 is arranged on the bed B. In detail, the attachment section 2 includes a base 21 and a housing support section 22. The base 21 is shaped like a flat plate and has a main surface 21a. The base 21 is arranged between the frame W and the mattress M (refer to FIG. 1) such that the main surface 21a is parallel to the upper surface of the frame W. As a result, the attachment section 2 is supported by the bed B. The mattress M preferably weighs enough to fix the base 21 using the mattress M and the frame W.

The housing support section 22 extends downward from the base 21. The housing support section 22 supports the main body 3. In the present embodiment, the housing support section 22 is shaped like a flat plate.

The main body 3 has a substantially rectangular parallelepiped-shaped housing 30. The housing 30 includes synthetic resin as a material, for example.

The suspension section 4 extends downward from the inside of the housing 30. The suspension section 4 includes a metal such as iron as a material, for example. The suspension section 4 has a base end 41 and a holding section 42.

The base end 41 is a rod-like member. The base end 41 extends in the up-and-down direction. The base end 41 penetrates a through hole formed in a lower wall of the housing 30. One end of the base end 41 is housed inside the housing 30, and the other end of the base end 41 is connected to the holding section 42.

The holding section 42 holds the urine collection container C. The holding section 42 suspends the urine collection container C. In the present embodiment, the holding section 42 is a hook. The hook has a substantial C-shape as viewed from a side, with a portion of a ring shape cut out. A gripping section H of the urine collection container C is hung from the hook. As a result, the urine collection container C is suspended.

The notification section 5 notifies of an operation state of the urine quantity measurement device 100. The notification section 5 is arranged on a side surface 30a of the housing 30. In the following, the embodiment is described on the assumption that the side of the housing 30 on which the side surface 30a is arranged is a "front side of the urine quantity measurement device 100" and an opposite side thereto is a "back side of the urine quantity measurement device 100". The embodiment is described also on the assumption that a left side of the housing 30 as viewed from the front side is a "left side of the urine quantity measurement device 100" and an opposite side thereto is a "right side of the urine quantity measurement device 100".

In the present embodiment, the notification section 5 includes two lamps. The two lamps are aligned in the up-and-down direction. A lighting state changes by each of the two lamps being lighted or flashing. A user can know the operation state of the urine quantity measurement device 100 by viewing a lighting state of each of the two lamps. The user can know, for example, that the urine quantity measurement device 100 is measuring a urine quantity by viewing the lighting state of each of the two lamps.

The operation section 6 receives operation for the urine quantity measurement device 100. The operation section 6 is arranged on the side surface 30a. In the present embodiment, the operation section 6 includes two operation buttons. The two operation buttons are aligned in the up-and-down direction. The user can operate the urine quantity measurement device 100 by pressing the two operation buttons. The user can, for example, start measurement of the urine quantity by pressing either of the two operation buttons. Alternatively, the user can end measurement of the urine quantity by pressing either of the two operation buttons.

Continuing, the configuration of the urine quantity measurement device 100 according to the first embodiment is further described with reference to FIG. 3. FIG. 3 is an exploded perspective view of the urine quantity measurement device 100 according to the first embodiment.

As illustrated in FIG. 3, the housing 30 has a main cover 31 and a sub cover 32. The main cover 31 is a box-shaped member with an open back surface. The sub cover 32 is a plate-shaped member. The upper end and the lower end of the sub cover 32 bend forwards.

The sub cover 32 is attachable to and detachable from the main cover 31. The sub cover 32 has four first through holes 321h, two second through holes 322h, four first fastening members B1, and two second fastening members B2.

The four first through holes 321h are provided on ends of the sub cover 32 in a left-right direction. In detail, two of the four first through holes 321h are provided on the left end of the sub cover 32. The two first through holes 321h provided on the left end of the sub cover 32 are aligned in the up-and-down direction. The remaining two of the four first through holes 321h are provided on the right end of the sub cover 32. The two first through holes 321h provided on the right end of the sub cover 32 are aligned in the up-and-down direction.

A first fastening member B 1 is inserted into each of the four first through holes 321h. The respective first fastening members B1 inserted into the four first through holes 321h are threaded into four fastening holes provided in the main cover 31. As a result, the sub cover 32 is fixed to the main cover 31. The sub cover 32 composes a back wall of the housing 30 when fixed to the main cover 31. It should be noted that an example of each first fastening member B1 is a screw, and an example of each fastening hole is a screw hole.

The two second through holes 322h are aligned in the left-right direction in the central part of the sub cover 32. A second fastening member B2 is inserted into each of the two second through holes 322h. The respective second fastening members B2 inserted into the two second through holes 322h are threaded into two fastening holes 22h provided in the housing support section 22. As a result, the sub cover 32 is fixed to the housing support section 22. In other words, the housing 30 is fixed to the housing support section 22. It should be noted that an example of each second fastening member B2 is a screw, and an example of each fastening hole 22h is a screw hole.

Continuing, a configuration of the attachment section 2 according to the first embodiment is further described with reference to FIG. 4. FIG. 4 is a diagram illustrating the configuration of the attachment section 2 according to the first embodiment.

As illustrated in FIG. 4, the attachment section 2 includes a first joint 23 in addition to the base 21 and the housing support section 22. The first joint 23 joins the base 21 to the housing support section 22. In the present embodiment, the first joint 23 restricts the posture of the urine quantity measurement device 100 such that a force acts in the vertical direction on the suspension section 4 described with reference to FIG. 2. It should be noted that the first joint 23 composes a first restricting section.

The first joint 23 includes an intermediate portion 230, a first axis portion 231, and a second axis portion 232. The intermediate portion 230 is shaped like a flat plate. The intermediate portion 230 is positioned between the base 21 and the housing support section 22. The first axis portion 231 is positioned at a connection location of the intermediate portion 230 where an edge thereof on a side of the housing support section 22 is connected to the housing support section 22. The second axis portion 232 is positioned at a connection location of the intermediate portion 230 where an edge thereof on a side of the base 21 is connected to the base 21. The first axis portion 231 and the second axis portion 232 extend in a direction intersecting with the up-and-down direction. Specifically, the first axis portion 231 and the second axis portion 232 extend in the left-right direction. In the present embodiment, the first axis portion 231 is formed by bending a part of the attachment section 2 where the housing support section 22 is connected to the intermediate portion 230, and the second axis portion 232 is formed by bending a part of the attachment section 2 where the intermediate portion 230 is connected to the base 21.

The first axis portion 231 allows the posture of the housing support section 22 to change relative to the posture of the intermediate portion 230. In detail, the housing support section 22 swings around the first axis portion 231.

The second axis portion 232 allows the posture of the intermediate portion 230 to change relative to the posture of the base 21. In detail, the intermediate portion 230 swings around the second axis portion 232.

The housing support section 22 is connected to the base 21 through the first joint 23. In detail, the housing support section 22 is connected to the base 21 through the first axis portion 231, the intermediate portion 230, and the second axis portion 232. Accordingly, the posture of the housing support section 22 is changeable relative to the base 21.

Continuing, a configuration of the suspension section 4 and the vicinity thereof according to the first embodiment is described with reference to FIG. 5. FIG. 5 is a diagram illustrating the suspension section 4 and the vicinity thereof according to the first embodiment. In detail, FIG. 5 illustrates a portion of the urine quantity measurement device 100 with the sub cover 32 removed.

As illustrated in FIG. 5, the urine quantity measurement device 100 further includes a sensor 7 and a controller 10. The sensor 7 and the controller 10 are housed in the housing 30.

As illustrated in FIG. 5, the sensor 7 outputs a signal indicating the weight of the urine collection container C described with reference to FIGS. 1 and 2 based on the force acting on the suspension section 4. The sensor 7 is a cantilever beam-type load cell, for example.

The sensor 7 includes a cantilever beam section 71 and a strain gauge 72. The cantilever beam section 71 includes a beam support section 711 and a first beam 712. The urine quantity measurement device 100 further includes a third fastening member B3.

The beam support section 711 is provided on an inner wall of the main cover 31. The beam support section 711 protrudes from the inner wall of the main cover 31 toward the inside of the main cover 31. In detail, the beam support section 711 protrudes upward from a lower wall of the main cover 31. In the present embodiment, the beam support section 711 is composed by the same material as the material which composes the main cover 31. The beam support section 711 includes synthetic resin as a material, for example.

The first beam 712 includes a metal such as iron as a material, for example. The first beam 712 is shaped like a flat plate. In the present embodiment, the first beam 712 extends in a horizontal direction. The first beam 712 is substantially rectangular as viewed from above. The first beam 712 has a first end 712a and a second end 712b. The first end 712a is fixed to the beam support section 711. In the present embodiment, the first end 712a is fixed to the upper surface of the beam support section 711. The second end 712b is movable in the up-and-down direction. In other words, the first beam 712 swings around, as a swinging center thereof, the location where the first beam 712 is fixed to the beam support section 711.

The base end 41 of the suspension section 4 is connected to the second end 712b. In the following, a part where the base end 41 is connected to the first beam 712 may be referred to as a "connection location 712c".

The base end 41 is fixed to the second end 712b so as to be orthogonal to the first beam 712. In the present embodiment, one end of the base end 41 penetrates a through hole provided in the first beam 712 and protrudes from the first beam 712. The one end of the base end 41 has a screw groove. The base end 41 of the suspension section 4 is fixed to the second end 712b by the nut-like third fastening member B3 being fastened to the screw groove provided on the one end of the base end 41.

When a force acts on the suspension section 4, the force acting on the suspension section 4 is transmitted to the first beam 712. As described with reference to FIG. 4, the posture of the urine quantity measurement device 100 is restricted such that a pulling force acts on the suspension section 4 in the vertical direction. Accordingly, the pulling force in the vertical direction acts on the second end 712b of the first beam 712 through the base end 41. When the pulling force in the vertical direction acts on the second end 712b of the first beam 712 through the base end 41, the first beam 712 is strained.

The strain gauge 72 detects a strain quantity (deformation quantity) of the first beam 712 and outputs a signal indicating the strain quantity. The strain quantity corresponds to the gross weight of the urine collection container C. Accordingly, the strain gauge 72 outputs a signal indicating the weight of the urine collection container C. It should be noted that the sensor 7 may further include an amplifier. The amplifier amplifies and outputs the signal output by the strain gauge 72. The signal output from the sensor 7 is sent to the controller 10.

The controller 10 controls the operation of each element of the urine quantity measurement device 100. The controller 10 includes a microcomputer, for example. Alternatively, the controller 10 includes an analog/digital (A/D) convertor, a processor such as a microprocessor, and at least one of read-only memory (ROM) and random-access memory (RAM). The A/D convertor converts the analog signal output from the sensor 7 into a digital signal.

The controller 10 generates output data based on the signal received from the sensor 7. In the present embodiment, the controller 10 generates output data at a prescribed gap. The prescribed gap is arbitrarily set by the user. The prescribed gap is one second, for example.

The output data indicates at least one of the gross weight of the urine collection container C and the urine quantity collected in the urine collection container C. In other words, the output data includes at least one of data indicating the gross weight of the urine collection container C and data indicating the urine quantity collected in the urine collection container C. The gross weight of the urine collection container C includes the weight of the urine collection container C and the weight of the urine collected in the urine collection container C. In the following, the data indicating the gross weight of the urine collection container C may be referred to as "gross weight data" and the data indicating the urine quantity collected in the urine collection container C may be referred to as "urine quantity data".

In the present embodiment, the controller 10 generates the urine quantity data based on the gross weight data. In other words, the output data includes the urine quantity data. In detail, the controller 10 generates the gross weight data based on the signal received from the sensor 7. The controller 10 then generates urine quantity data by dividing the gross weight indicated by the gross weight data by a prescribed value. The prescribed value is preset by the user. The prescribed value is set based on the specific gravity of typical urine. It should be noted that the prescribed value may be set based on the specific gravity of urine taken from the person P illustrated in FIG. 1. The prescribed value is "1.0", for example.

Continuing, the configuration of the urine quantity measurement device 100 according to the first embodiment is further described with reference to FIG. 6. FIG. 6 is a block diagram illustrating the configuration of the urine quantity measurement device 100 according to the first embodiment.

As illustrated in FIG. 6, the urine quantity measurement device 100 further includes a power source 8 and a communication section 9. The communication section 9 is an example of an output section.

The power source 8 supplies power to the notification section 5, the operation section 6, the sensor 7, the communication section 9, and the controller 10. A dry cell is attached to the power source 8, for example. Alternatively, the power source 8 may be a battery which stores power. The battery is charged with power provided from outside through an AC adapter.

The communication section 9 sends and receives data to and from an electronic device equipped with a communication device using the same communication method as the communication section 9. In the present embodiment, the communication section 9 is connected to an external terminal 200 through a network N such as a wireless local area network (LAN), for example. The external terminal 200 is a mobile terminal such as a smartphone or a tablet computer, for example. The communication section 9 is a LAN adapter such as a wireless LAN adapter, for example. The communication section 9 sends the output data generated by the controller 10 to the external terminal 200. In the present embodiment, the communication section 9 sends the urine quantity data to the external terminal 200.

The external terminal 200 includes a terminal communication section 201, a touch panel 202, and a terminal controller 210.

The terminal communication section 201 sends and receives data to and from an electronic device equipped with a communication device using the same communication method as the terminal communication section 201. In the present embodiment, the terminal communication section 201 sends and receives data to and from the communication section 9 of the urine quantity measurement device 100. The terminal communication section 201 is a LAN adapter such as a wireless LAN adapter, for example.

The touch panel 202 includes a display and a touch sensor.

The display displays various screens. The display is a liquid-crystal display, for example. It should be noted that the display may alternatively be an organic electroluminescent (EL) display.

The touch sensor receives operation (touch) by the user. The touch sensor outputs a signal indicating a touched position when receiving a touch by the user. The touch sensor is a resistive touch sensor, for example. It should be noted that the touch sensor may alternatively be an electrostatic capacitive touch sensor.

The terminal controller 210 includes a processor such as a central processing unit (CPU), for example. The terminal controller 210 also includes at least one of ROM and RAM. The at least one of ROM and RAM compose a storage area. In the present embodiment, the storage area of the terminal controller 210 stores a urine quantity measurement application. The storage area of the terminal controller 210 also stores the output data. It should be noted that the external terminal 200 may further include a storage device such as a hard disk drive (HDD) as a component which composes the storage area.

The terminal controller 210 causes the touch panel 202 to display a urine quantity measurement screen based on the output data when the terminal communication section 201 receives the output data from the communication section 9. The urine quantity measurement screen is described with reference to FIG. 7. The urine quantity measurement screen is displayed on the touch panel 202 by the terminal controller 210 executing the urine quantity measurement application.

Continuing, a urine quantity measurement screen G according to the first embodiment is described with reference to FIGS. 7 and 8. FIGS. 7 and 8 are diagrams illustrating examples of the urine quantity measurement screen G according to the first embodiment. In the present embodiment, the urine quantity measurement screen G includes a first urine quantity measurement screen G1 (refer to FIG. 7) and a second urine quantity measurement screen G2 (refer to FIG. 8).

As illustrated in FIG. 7, the first urine quantity measurement screen G1 includes a first display field d1, a second display field d2, a third display field d3, a fourth display field d4, a fifth display field d5, a sixth display field d6, a seventh display field d7, and a first button b1.

The first display field d1 shows the current time. In the example illustrated in FIG. 7, the first display field d1 shows that the current time is "16:20".

The second display field d2 shows the current date and day of the week. In the example illustrated in FIG. 7, the second display field d2 shows that the current date is "9/21" and the current day of the week is "Friday".

The third display field d3 shows an interval. The interval indicates a gap at which a urine quantity displayed in the fourth display field d4 is updated. In the example illustrated in FIG. 7, the third display field d3 shows that the urine quantity shown in the fourth display field d4 is updated at a five-minute gap.

The fourth display field d4 shows the most recently measured urine quantity. In the present embodiment, the terminal controller 210 (refer to FIG. 6) causes the fourth display field d4 to display the most recently measured urine quantity based on the most recent output data stored in the storage area and the output data stored in the storage area five minutes (the interval) earlier. The urine quantity displayed in the fourth display field d4 is updated at a five-minute gap. In the example illustrated in FIG. 7, the fourth display field d4 shows that the most recent urine quantity is "5 ml".

The fifth display field d5 shows a previously measured urine quantity. The urine quantity displayed in the fourth display field d4 before updating is displayed in the fifth display field d5. In the example illustrated in FIG. 7, the fifth display field d5 shows that the previously measured urine quantity is "7 ml".

The sixth display field d6 shows a total urine quantity. The total urine quantity indicates a total quantity of urine quantities measured from the beginning of urine quantity measurement until the current time. In the present embodiment, the total urine quantity is a urine quantity indicating the most recent output data. In the example illustrated in FIG. 7, the sixth display field d6 shows that the total urine quantity is "350 ml".

The seventh display field d7 shows a total measurement time. The total measurement time indicates elapsed time from the beginning of urine measurement to the current time.

The first button b1 is a button for selecting an operating mode of the urine quantity measurement application. The first button b1 also indicates the current operating mode of the urine quantity measurement application. In the example illustrated in FIG. 7, the first button b1 indicates that the operating mode of the urine measurement application is a "record mode".

In the record mode, the urine measurement application causes the touch panel 202 to display the first urine quantity measurement screen G1 which displays the measured urine quantity.

When the user touches the touch panel 202 over the first button b1, the first button b1 is selected. When the first button b1 is selected, the second urine quantity measurement screen G2 is displayed on the touch panel 202 as illustrated in FIG. 8.

The second urine quantity measurement screen G2 includes a urine quantity list L1, an eighth display field d8, a second button b2, and a third button b3.

The urine quantity list L1 displays urine quantities measured at different times in a list. In the example illustrated in FIG. 8, the urine quantity list L1 displays the urine quantities for 30 cases in a list. In detail, the urine quantity list L1 illustrated in FIG. 8 displays urine quantities measured at one-minute gaps from "20:11" to "20:40".

The eighth display field d8 shows the gap at which the urine quantities are measured. In the example illustrated in FIG. 8, the eighth display field d8 shows that urine quantities measured at one-minute gaps are displayed in a list in the urine quantity list L1. It should be noted that in the present embodiment, the gap shown by the eighth display field d8 matches the prescribed gap described with reference to FIG. 5.

When the user touches the touch panel 202 over the second button b2, the list of urine quantities displayed in the urine quantity list L1 is updated. In detail, a list of urine quantities measured before the time when the currently displayed urine quantity was measured is displayed in the urine quantity list L1. In the second urine quantity measurement screen G2 illustrated in FIG. 8, a list of urine quantities measured at 20:10 or earlier is displayed in the urine quantity list L1 when the second button b2 is touched.

Furthermore, when the user touches the touch panel 202 over the third button b3, the list of urine quantities displayed in the urine quantity list L1 is changed. In detail, a list of urine quantities measured after the time when the currently displayed urine quantity was measured is displayed in the urine quantity list L1. In the second urine quantity measurement screen G2 illustrated in FIG. 8, a list of urine quantities measured at 20:41 or later is displayed in the urine quantity list L1 when the second button b2 is touched.

Furthermore, when the user touches any position on the second urine quantity measurement screen G2 other than the second button b2 or the third button b3, the screen displayed on the touch panel 202 switches. In detail, the screen displayed on the touch panel 202 switches from the second urine quantity measurement screen G2 to the first urine quantity measurement screen G1.

It should be noted that the operating mode of the urine measurement application includes a "setting mode", for example, in addition to the "record mode". In the setting mode, the urine measurement application causes the touch panel 202 to display a setting screen for setting the prescribed gap described with reference to FIG. 5, for example. The urine measurement application also causes display of a setting screen for setting the interval at which the urine quantities displayed in the fourth display field d4 and the fifth display field d5 are updated. In other words, the urine measurement application causes display of a setting screen for setting the interval displayed in the third display field d3. The user can change the prescribed gap or the interval in the setting screen.

The first embodiment is described above. According to the present embodiment, the urine quantity measurement device 100 is to be used while attached to the bed B. Accordingly, no instrument for attachment of the urine quantity measurement device 100 need be arranged at the side of the bed B. Therefore, a urine quantity can be measured without need of providing an arrangement space at the side of the bed B in which an instrument is to be arranged.

Furthermore, according to the present embodiment, the shape of the attachment section 2 can be changed according to the shape of the frame W of the bed B by the first joint 23. In other words, the posture of the housing support section 22 relative to the base 21 can be changed according to the shape of the frame W of the bed B. In detail, the posture of the housing support section 22 is restricted such that the direction of the pulling force from the urine collection container C is in a constant direction (vertical direction). In other words, the postures of the main body 3 and the holding section 42 are restricted such that the direction of the pulling force from the urine collection container C is a constant direction. As a result, the direction of the pulling force acting on the sensor 7 is kept constant (in the vertical direction). Therefore, the sensor 7 can stably measure the weight of the urine collection container C. Accordingly, the precision of the result of measurement of the urine quantity is improved.

Also in the present embodiment, the housing support section 22 changes posture by pivoting around the first axis portion 231 and the second axis portion 232 as pivotal centers. In other words, the housing support section 22 changes posture by pivoting around two axes as pivotal centers. The degree of freedom of the posture of the housing support section 22 increases as the number of axes increases. That is, compared to a case where the number of axes is one, the posture of the housing support section 22 is changed more easily according to the shape of the frame W of the bed B. Accordingly, the precision of the result of measurement of the urine quantity improves.

Furthermore, urine quantities are often measured by a nurse or a doctor visually confirming a scale provided on the urine collection container C. As such, the precision of the result of measurement of a urine quantity is not stable. According to the present embodiment, the urine quantity measurement device 100 measures the urine quantity by measuring the gross weight of the urine collection container C. Therefore, the precision of the result of measurement of the urine quantity is improved compared to a case where a nurse or a doctor visually confirms. Also according to the present embodiment, labor of a nurse or a doctor can be decreased.

Furthermore, when a plurality of base ends 41 are connected to the first beam 712 and forces act on a plurality of locations on the first beam 712, there may be variation in the strain occurring in the first beam 712. When there is variation in the strain occurring in the first beam 712, the precision of the result of measurement of the urine quantity decreases. According to the present embodiment, the sensor 7 only has one connection location 712c. Accordingly, variation in the strain occurring in the first beam 712 is inhibited. As a result, the precision of the result of measurement of the urine quantity is improved compared to a case where the sensor 7 has a plurality of connection locations 712c. However, the sensor 7 may have a plurality of connection locations 712c.

It should be noted that the present embodiment describes a configuration in which the sub cover 32 is fixed to the housing support section 22 by the second fastening members B2, but the configuration in which the sub cover 32 is fixed to the housing support section 22 is not limited as such. FIG. 9 is a diagram illustrating an alternative example of the housing support section 22 and the housing 30 according to the first embodiment. As illustrated in FIG. 9, the sub cover 32 may have a hook hole 30h and the housing support section 22 may include a hook section 22f. The sub cover 32 may be fixed to the housing support section 22 by the hook hole 30h being hung on the hook section 22f.

The present embodiment also describes a configuration in which the first joint 23 includes two axis portions, but the first joint 23 may include one axis portion or three or more axis portions. Alternatively, the first joint 23 may not include the first axis portion 231 or the second axis portion 232. In this case, the base 21 and the housing support section 22 are fixed by welding, for example.

The present embodiment also describes a configuration in which the attachment section 2 includes paper as a material. However, the attachment section 2 is not limited to a configuration of including paper as a material. The attachment section 2 need only be capable of being arranged on the bed B to support the housing 30, and may include metal as a material, for example.

Also in the present embodiment, the attachment section 2 is made of a single plate-shaped member and the first joint 23 is formed by bending the attachment section 2. However, the first joint 23 is not limited to a case of being formed by bending the attachment section 2. FIG. 10A is a diagram illustrating a first alternative example of the first joint 23 according to the first embodiment. FIG. 10B is a diagram illustrating a second alternative example of the first joint 23 according to the first embodiment. As illustrated in FIG. 10A, the first joint 23 may be composed by a hinge, for example. Alternatively, as illustrated in FIG. 10B, the first joint 23 may be composed by a ring-shaped fastener, for example.

The present embodiment also describes a case in which the attachment section 2 is a flat plate. However, the attachment section 2 may have an opening as illustrated in FIG. 11A. FIG. 11A is a diagram illustrating a first alternative example of the attachment section 2 according to the first embodiment.

The present embodiment also describes a case in which the attachment section 2 is formed by bending a single flat plate. However, the attachment section 2 may be formed by bending a linear member as illustrated in FIG. 11B. FIG. 11B is a diagram illustrating a second alternative example of the attachment section 2 according to the first embodiment.

The present embodiment also describes a case in which the attachment section 2 is attached to the bed B by being arranged between the mattress M and the frame W, but the attachment section 2 need only be arranged on the bed B, and may be a linear member such as a cord, for example. In this case, the attachment section 2 may be arranged in a railing provided in the bed B, for example.

The present embodiment also describes a case in which the suspension section 4 includes metal as a material. However, the suspension section 4 may include for example at least one of fiber, synthetic resin, or metal as a material. Specifically, the suspension section 4 may be a cord, a cable tie, or VELCRO (registered Japanese trademark). Alternatively, the suspension section 4 may be composed by a combination of two or more of a cord, a cable tie, and VELCRO (registered Japanese trademark).

The present embodiment also describes a case in which the shape of a hook is a C-shape when viewed from a side, but the shape of a hook is not limited as such. The hook may be any shape as long as it is capable of suspending the urine collection container C, and may be a substantial U-shape as viewed from a side, for example.

The present embodiment also describes a configuration in which the holding section 42 is a hook, but the holding section 42 may be any structure as long as it is capable of suspending the urine collection container C, and the holding section 42 may be a clip which grips the urine collection container C.

The present embodiment also describes a case in which the first beam 712 of the sensor 7 is substantially rectangular as viewed from above, but the first beam 712 is not limited to a case of being substantially rectangular as viewed from above.

The present embodiment also describes a case in which the communication section 9 is an example of an output section, but the output section may be the notification section 5, for example. The notification section 5 may output data indicating a urine quantity by for example changing the lighting state of the lamps.

The present embodiment also describes a configuration in which the notification section 5 includes two lamps, but the number of lamps included in the notification section 5 may be one or three or more.

The present embodiment also describes a configuration in which the operation section 6 includes two operation buttons, but the number of operation buttons included in the operation section 6 may be one or three or more.

The present embodiment also describes a case in which the interval displayed in the third display field d3 of the first urine quantity measurement screen G1 does not match the gap at which the output data is sent from the urine quantity measurement device 100. However, the interval displayed in the third display field d3 of the first urine quantity measurement screen G1 may match the gap at which the output data is sent from the urine quantity measurement device 100.

The present embodiment also describes a case in which the measurement gap of the urine quantities displayed in the urine quantity list L1 matches the prescribed gap, but the measurement gap of the urine quantities displayed in the urine quantity list L1 may differ from the prescribed gap.

The present embodiment also describes a case in which the controller 10 of the urine quantity measurement device 100 generates urine quantity data as the output data and sends the urine quantity data to the external terminal 200. However, the controller 10 of the urine quantity measurement device 100 may generate gross weight data as the output data and the gross weight data may be sent to the external terminal 200. In this case, the terminal controller 210 of the external terminal 200 calculates a urine quantity based on the gross weight data. The terminal controller 210 generates the urine quantity data by dividing the gross weight indicated by the gross weight data by a prescribed value according to a method similar to the method of calculating a urine quantity described with reference to FIG. 5.

The present embodiment also describes a case in which the controller 10 of the urine quantity measurement device 100 generates the output data at a prescribed gap. However, the controller 10 may continuously generate the output data while the signal indicating the gross weight of the urine collection container C is being received. In this case, the controller 10 controls a timing at which the communication section 9 sends the output data. The controller 10 may also cause the sensor 7 to detect a strain quantity at a prescribed gap.

### [Second Embodiment]

Continuing, a configuration of the urine quantity measurement device 100 according to a second embodiment is described with reference to FIG. 12. The second embodiment differs from the first embodiment in the configuration of the suspension section 4. In the following, items of the second embodiment which differ from the first embodiment are described and description of parts shared with the first embodiment is omitted.

FIG. 12 is a diagram illustrating a configuration of the suspension section 4 according to the second embodiment. As illustrated in FIG. 12, the suspension section 4 further includes a second joint 43 in addition to the base end 41 and the holding section 42. The second joint 43 joins the base end 41 to the holding section 42. In the present embodiment, the second joint 43 restricts the posture of the suspension section 4 such that the direction of the pulling force from the urine collection container C is in the vertical direction. The second joint 43 composes a second restricting section.

The second joint 43 includes a universal coupling 431. The universal coupling 431 has two axes. Due to provision of the universal coupling 431, the holding section 42 is swingable relative to the base end 41 in the front-back direction and the left-right direction. For example, the universal coupling 431 flexibly changes a joint angle α between the base end 41 and the holding section 42 in the front-back direction. In other words, the second joint 43 changes the posture of the holding section 42 relative to the base end 41.

The second embodiment 2 is described above. According to the present embodiment, the suspension section 4 includes a universal coupling 431. The universal coupling 431 changes the posture of the holding section 42 relative to the base end 41. In detail, the universal coupling 431 keeps the direction of the pulling force acting on the first beam 712 constant (in the vertical direction) even in a case in which the posture of the holding section 42 is inclined relative to the vertical direction. As a result, the precision of the result of measurement of a urine quantity increases.

### [Third Embodiment]

Continuing, a configuration of the urine quantity measurement device 100 according to a third embodiment is described with reference to FIG. 13. The third embodiment differs from the first embodiment and the second embodiment in the configuration of the suspension section 4. In the following, items of the third embodiment which differ from the first embodiment and the second embodiment are described and description of parts shared with the first embodiment and the second embodiment is omitted.

FIG. 13 is a diagram illustrating the configuration of the urine quantity measurement device 100 according to the third embodiment. In the present embodiment as illustrated in FIG. 13, the suspension section 4 has two holding sections 42.

In the present embodiment, the second joint 43 includes a second beam 44. The second beam 44 is a rod-like member. In the present embodiment, the second beam 44 extends in a direction matching the longitudinal direction of the housing 30. Specifically, the second beam 44 extends in a direction intersecting with the vertical direction. The second beam 44 extends in the horizontal direction.

The second beam 44 is arranged beneath the housing 30. The second beam 44 is arranged between the base end 41 and the holding sections 42. In detail, the lower end of the base end 41 is connected to a central portion of the second beam 44 in the longitudinal direction of the second beam 44. The two holding sections 42 are connected to parts of the second beam 44 which are away from the part where the base end 41 is connected. In detail, the two holding sections 42 are connected to positions on the second beam 44 which are separated from the part where the base end 41 is connected by equal distances. In the present embodiment, the two holding sections 42 are connected to two respective ends of the second beam 44 in the longitudinal direction of the second beam 44. The second beam 44 transmits forces acting on the two holding sections 42 to the base end 41. As described with reference to FIG. 5, the base end 41 is connected to the first beam 712 at the connection location 712c, and transmits the forces acting on the two holding sections 42 to the first beam 712.

The third embodiment is described above. According to the present embodiment, the urine quantity measurement device 100 can suspend one urine collection container C by two holding sections 42. Therefore, the urine collection container C can be suspended in a stable posture. As a result, the pulling force from the urine collection container C can be caused to act on the sensor 7 in the vertical direction. Accordingly, the precision of the result of measurement of the urine quantity improves.

Also in the present embodiment, the forces acting on the two holding sections 42 are combined in the second beam 44 and transmitted to the base end 41. The base end 41 is connected to the first beam 712 at the connection location 712c. Accordingly, variation in the strain occurring in the first beam 712 can be inhibited. Therefore, the precision of the result of measurement of the urine quantity improves.

Also in the present embodiment, the two holding sections 42 are connected to the second beam 44 at positions which are separated from the part where the base end 41 is connected by equal distances. Accordingly, the pulling forces caused to act by the urine collection container C can be uniformly distributed on the two holding sections 42. Therefore, the urine collection container C can be suspended in a stable posture. As a result, the precision of the result of measurement of a urine quantity improves.

It should be noted that the present embodiment describes a configuration in which the second beam 44 is arranged below the housing 30, but the position at which the second beam 44 is arranged is not limited to below the housing 30. FIG. 14 is a diagram illustrating a first alternative example of the suspension section 4 according to the third embodiment of the present invention.

As illustrated in FIG. 14, the second beam 44 may be arranged inside the housing 30. In this case, two through holes are formed in a lower wall of the housing 30. The base end of each of the two holding sections 42 penetrates a respective through hole, and the tip of each of the two holding sections 42 is arranged outside the housing 30. The base ends of the two holding sections 42 are connected to the second beam 44. In detail, the two holding sections 42 are connected to the second beam 44 such that the forces acting on the two holding sections 42 are equal.

Furthermore, the second beam 44 may be freely lengthened and shortened in the longitudinal direction. For example, as illustrated in FIG. 15, the second beam 44 may include a beam housing 440, a first lengthening and shortening section 441, a second lengthening and shortening section 442, and a gear 443. FIG. 15 is a diagram illustrating a second alternative example of the suspension section 4 according to the third embodiment.

The beam housing 440 is bottomless and cylindrical, and extends in the longitudinal direction of the second beam 44. A portion of the first lengthening and shortening section 441, a portion of the second lengthening and shortening section 442, and the gear 443 are housed inside the beam housing 440. The gear 443 is pivotally supported by the beam housing 440. The first lengthening and shortening section 441 and the second lengthening and shortening section 442 are opposite to each other with the gear 443 therebetween. The beam housing 440 movably supports the first lengthening and shortening section 441 and the second lengthening and shortening section 442 in the longitudinal direction of the second beam 44. A plurality of grooves are formed in a surface of the first lengthening and shortening section 441 on a side of the gear 443. The grooves mesh with the teeth of the gear 443. Similarly, a plurality of grooves are formed in a surface of the second lengthening and shortening section 442 on a side of the gear 443. The grooves mesh with the teeth of the gear 443. The grooves of the first lengthening and shortening section 441 and the second lengthening and shortening section are aligned with each other in the longitudinal direction of the second beam 44. In response to the first lengthening and shortening section 441 being moved in the longitudinal direction of the second beam 44, the second lengthening and shortening section 442 also moves in the longitudinal direction of the second beam 44. Similarly, in response to the second lengthening and shortening section 442 being moved in the longitudinal direction of the second beam 44, the first lengthening and shortening section 441 also moves in the longitudinal direction of the second beam 44. Specifically, the first lengthening and shortening section 441 and the second lengthening and shortening section 442 move by the same distance in mutually opposite directions. As a result, by one of the first lengthening and shortening section 441 and the second lengthening and shortening section being moved, the length of respective parts of the first lengthening and shortening section 441 and the second lengthening and shortening section 442 housed in the beam housing 440 changes. In other words, the second beam 44 lengthens or shortens. Due to the second beam 44 lengthening or shortening, the distance between of the holding sections 42 can be adjusted to match the size of the gripping section H of the urine collection container C. Therefore, the urine collection container C can be stably suspended.

The present embodiment also describes a case in which the number of holding sections 42 is two, but the number of holding sections 42 is not limited to two. The number of holding sections 42 may be three or more.

Also in the present embodiment, a configuration is described in which the second joint 43 includes the second beam 44, but the second joint 43 may further include the universal coupling 431 described with reference to FIG. 12. The universal coupling 431 is provided between the second beam 44 and the base end 41.

### [Fourth Embodiment]

Continuing, a configuration of the urine quantity measurement device 100 according to a fourth embodiment is described with reference to FIG. 16A. The fourth embodiment differs from the first to third embodiments in the configuration of the suspension section 4. In the following, items of the fourth embodiment which differ from the first to third embodiments are described and description of parts shared with the first to third embodiments is omitted.

FIG. 16A is a diagram illustrating a suspension section 4 according to the fourth embodiment. As illustrated in FIG. 16A, the suspension section 4 of the present embodiment further includes an adapter 400. The adapter 400 is applicable to various types of the urine collection container C.

The adapter 400 freely changes (deforms) in outer shape. The adapter 400 is composed by a wire, for example. The adapter 400 includes a connecting section 401, a shaft 402, two branching portions 403, and two locking portions 404. In the present embodiment, the connecting section 401, the shaft 402, and the two branching portions 403 function as the second joint 43.

The connecting section 401 is connected to the base end 41. The connecting section 401 is hung from a hook of the base end 41. The connecting section 401 is ring-shaped, for example.

The shaft 402 is connected to the connecting section 401 and the two branching portions 403. The shaft 402 is connected to a lower end of the connecting section 401 and extends downward. The two branching portions 403 extend from a tip of the shaft 402 in different directions. The distance between the two branching portions 403 can be changed by deforming the adapter 400. It should be noted that the two branching portions 403 function as the second beam 44.

The two locking portions 404 are provided on respective tips of the two branching portions 403. The gripping section H of the urine collection container C is hung on the two locking portions 404. It should be noted that each locking portion 404 functions as the holding section 42.

The fourth embodiment is described above. According to the present embodiment, the adapter 400 can stably suspend the urine collection container C regardless of the shape of the urine collection container C by changing the distance between the two branching portions 403.

It should be noted that the present embodiment describes a case in which the number of locking portions 404 is two. However, the number of locking portions 404 may be any number which enables stable suspension of the urine collection container C and is not limited to two. For example, the number of the locking portions 404 may be four as illustrated in FIG. 16B. FIG. 16B is a diagram illustrating an alternative example of the suspension section 4 according to the fourth embodiment.

Furthermore, a configuration is described in which each locking portion 404 is provided on the tip of a corresponding one of the branching portions 403, but each locking portion 404 may for example be provided between the tip and the base end of a corresponding one of the branching portions 403 as illustrated in FIG. 16B. In this case, each locking portion 404 is composed by a recess formed by bending a corresponding one of the two branching portions 403. It should be noted that each recess of the two branching portions 403 is symmetrically provided with the shaft 402 as an axis. Therefore, the suspension section 4 can stably suspend the urine collection container C.

Embodiments of the present invention are described above with reference to the accompanying drawings (FIGS. 1 to 16B). However, the present invention is not limited to the above embodiments and may be implemented in various manners within a scope not departing from the gist thereof. Furthermore, aspects such as shape, number, and material illustrated in the above embodiments are examples and not particular limitations. Various alterations are possible within a scope not substantially departing from the effects of the present invention.

For example, the embodiments of the present invention describe a case in which the sensor 7 is a load cell, but the sensor 7 may be a pressure sensor, for example.

Furthermore, the items described in each of the first to fourth embodiments may be appropriately combined. For example, items described in the second embodiment may be combined with items described in the third embodiment.

### [INDUSTRIAL APPLICABILITY]

The present invention is applicable to the field of medicine, for example. The present invention is also applicable to the field of caregiving, for example.

### [REFERENCE SIGNS LIST]

- 1: Urine quantity measurement device
- 2: Attachment section
- 3: Main body
- 4: Suspension section
- 7: Sensor
- 9: Communication section
- 10: Controller
- 21: Base
- 22: Housing support section
- 23: First joint (first restricting section)
- 30: Housing
- 41: Base end
- 42: Holding section
- 43: Second joint (second restricting section)
- 44: Beam
- B: Bed
- C: Urine collection container
- P: Person

## Claims

1. A urine quantity measurement device comprising:
a suspension section from which a urine collection container is suspended, the urine collection container being for collecting urine from a person lying on a bed;
a sensor configured to output a signal indicating a weight of the urine collection container based on a force acting on the suspension section from the urine collection container;
a controller configured to generate output data based on the signal;
a housing which houses the sensor and the controller; and
an attachment section arranged on the bed and configured to support the housing, wherein
the output data indicates the weight of the urine collection container or a quantity of the urine collected in the urine collection container.

2. The urine quantity measurement device according to claim 1, further comprising
a restricting section configured to restrict a posture of the urine quantity measurement device such that a pulling force from the urine collection container is caused to act on the sensor in a vertical direction.

3. The urine quantity measurement device according to claim 2, wherein
the attachment section includes:
a base arranged on the bed;
a housing support section which supports the housing; and
a first joint which joins the base to the housing support section.

4. The urine quantity measurement device according to claim 3, wherein
the restricting section includes a first restricting section,
the first joint changes a posture of the housing support section relative to the base, and
the first restricting section is composed by the first joint.

5. The urine quantity measurement device according to any one of claims 2 to 4, wherein
the suspension section includes:
a base end connected to the sensor;
a holding section from which the urine collection container is suspended; and
a second joint which joins the base end to the holding section.

6. The urine quantity measurement device according to claim 5, wherein
the restricting section includes a second restricting section,
the second joint changes a posture of the holding section relative to the base end, and
the second restricting section is composed by the second joint.

7. The urine quantity measurement device according to claims 5 or 6, wherein
the sensor includes a beam extending in a direction intersecting with the vertical direction,
the base end extends from the beam in a direction intersecting with the direction in which the beam extends, and
the holding section is provided on an opposite side of the beam from a side to which the base end is connected.

8. The urine quantity measurement device according to any one of claims 5 to 7, wherein
the holding section is provided as a plurality of holding sections.

9. The urine quantity measurement device according to claim 8, wherein
the sensor includes a first beam extending in a direction intersecting with the vertical direction,
the second joint includes a second beam,
the base end extends from the first beam in a direction intersecting with the direction in which the first beam extends,
the second beam is provided on an opposite side of the first beam from a side to which the base end is connected, and
the plurality of holding sections are provided in positions on the second beam at which forces acting from the urine collection container are uniform.

10. A urine quantity measurement device comprising:
a suspension section from which a urine collection container is suspended, the urine collection container being for collecting urine from a person lying on a bed;
a sensor configured to output a signal indicating a weight of the urine collection container based on a force acting on the suspension section from the urine collection container;
a controller configured to generate output data based on the signal;
a housing which houses the sensor and the controller;
an attachment section arranged on the bed and configured to support the housing; and
an output section configured to output the output data, wherein
the sensor includes a beam extending in a direction intersecting with a vertical direction,
the suspension section includes a base end and a holding section,
the base end extends from the beam in a direction intersecting with the direction in which the beam extends,
the holding section is provided on an opposite side of the beam from a side to which the base end is connected,
the urine collection container is suspended by the holding section, and
the output data indicates the weight of the urine collection container or a quantity of the urine collected in the urine collection container.

11. A urine quantity measurement device comprising:
a suspension section from which a urine collection container is suspended, the urine collection container being for collecting urine from a person lying on a bed;
a sensor configured to output a signal indicating a weight of the urine collection container based on a force acting on the suspension section from the urine collection container;
a controller configured to generate output data based on the signal;
a housing which houses the sensor and the controller;
an attachment section arranged on the bed and configured to support the housing; and
an output section configured to output the output data, wherein
the sensor includes a first beam extending in a direction intersecting with a vertical direction,
the suspension section includes a base end, a second beam, and a plurality of holding sections,
the base end extends from the first beam in a direction intersecting with the direction in which the first beam extends,
the second beam is provided on an opposite side of the first beam from a side to which the base end is connected,
the plurality of holding sections are provided in positions on the second beam at which forces acting from the urine collection container are uniform,
the urine collection container is suspended by the plurality of holding sections, and
the output data indicates the weight of the urine collection container or a quantity of the urine collected in the urine collection container.
